# EUROPEAN PATENT APPLICATION

(11) **EP 4 265 255 A1**
(43) Date of publication of application: **25.10.2023**
(21) Application number: 21911473.3
(22) Date of filing: 21.12.2021
(51) Int. Cl.: A61K 31/5377, A61K 9/00, A61P 3/04, A61P 3/10, A61P 29/00, A61P 15/10

(54) **USE AS SELECTIVE AGONIST OF MALANOCORTIN-4 RECEPTOR**

(30) Priority: 22.12.2020 KR 20200180505
(71) Applicant: Lg Chem, Ltd., Seoul 07336 (KR)
(72) Inventor: PARK, Hee Dong, Seoul 07796 (KR); YEO, Su Jin, Seoul 07796 (KR); PARK, Hyun Seo, Seoul 07796 (KR); PARK, Jin Sook, Seoul 07796 (KR); AHN, Hye Won, Seoul 07796 (KR)
(74) Representative: Hoffmann Eitle
(86) International application number: PCT/KR2021/019500
(87) International publication number: WO 2022/139406

(57) **Abstract**

The present invention relates to use of a compound of chemical formula 1 or a pharmaceutically acceptable salt thereof as a selective agonist of the melanocortin-4-receptor (MC4R).

## Description

### TECHNICAL FIELD

The present invention relates to the use of a compound of the following Formula 1 or a pharmaceutically acceptable salt thereof as a selective agonist for melanocortin-4 receptor (MC4R): 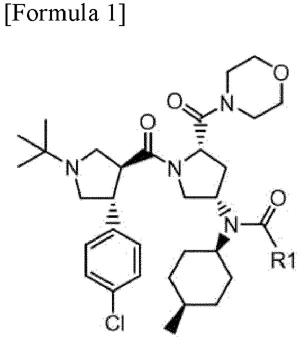 wherein R1 is C₂-C₅ alkyl.

### BACKGROUND ART

Melanocortin receptor (MCR) is a type of G-protein coupled receptor (GPCR), and the main role of G-protein is to activate secondary messengers and regulate the response of cells to many physiological stimuli through signal transduction. Until now, five subtypes of melanocortin receptor have been identified. MC1R is mainly expressed in melanocytes and macrophages, and determines the color of skin and hair by regulating melanin pigment in melanocytes. MC2R is expressed in the adrenal gland and adipose tissue, and the mediating function of adrenal hormone secretion regulation by adrenocorticotropic hormone in the adrenal gland is well known. MC3R, MC4R and MC5R are expressed not only in nerve terminals but also in the brain, and thus it is understood that they mediate central nerve actions by melanocortin peptides, which are expressed as effects on behavior, learning, memory, appetite, and generation and regeneration of nerves. Until now, MC3R is known to be involved in erectile dysfunction and inflammatory response, and MC4R is known to be involved in obesity and diabetes, and studies on the specificity of actions of each receptor are being actively carried out (MacNeil DJ et al., Eur J Pharmacol 2002, 450, 93). As a result, it was found that MC4R is deeply involved in genetic studies in people with obesity, and it was demonstrated that this receptor plays an important role in appetite regulation by showing that knockout mice in which MC4R was removed develop obesity by overeating (Lu D, Willard D et al., Nature 1994, 371(6500), 799; Huszar D et al., Cell 1997, 88(1), 131; Hinney A et al., J Clin Endocrinol Metab 1990, 84(4), 1483).

Among melanocortin receptors, MC4R is known to be involved in energy metabolism and body weight control. In the case of other MCR subtypes, because they are involved in the regulation of various *in vivo* functions such as skin pigmentation, energy homeostasis and exocrine function, it is very important to secure selectivity of MC4R agonist compounds for MC4R in preventing side effects which may occur in the future. Specifically, MC1R is mainly expressed in skin cells and is known to be involved in skin pigmentation through melanin pigment activity. The side effects of skin pigmentation appearing in existing non-selective agonists for melanocortin receptors may be mainly caused by MC1R-stimulating ability and intradermal drug accumulation. As such, among the existing compounds, the present inventors tried to discover a compound that can exhibit selectivity for MC4R by understanding the relationship between the structure and activity that can selectively activate MC4R. (Wikberg et al, Eur. J. Pharmacol 1999, 375, 295-310; Wikberg, et al., Pharm Res 2000, 42 (5) 393-420; Douglas et al., Eur J Pharm 2002, 450, 93-109; O'Rahilly et al., Nature Med 2004, 10, 351-352.)

### DISCLOSURE OF INVENTION

### TECHNICAL PROBLEM

The present invention is intended to provide the use of a compound of the following Formula 1 or a pharmaceutically acceptable salt thereof as a selective agonist for melanocortin-4 receptor (MC4R): 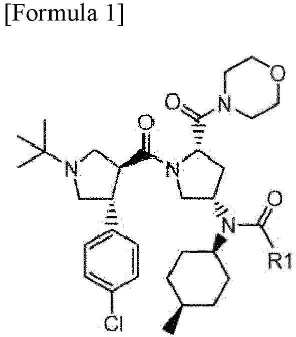 wherein R1 is C₂-C₅ alkyl.

### SOLUTION TO PROBLEM

The present invention provides a medicament for the prevention or treatment of a disease associated with melanocortin receptor having a selective agonistic effect for melanocortin-4 receptor, which comprises a therapeutically effective amount of a compound of the following Formula 1 or a pharmaceutically acceptable salt thereof: 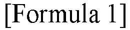 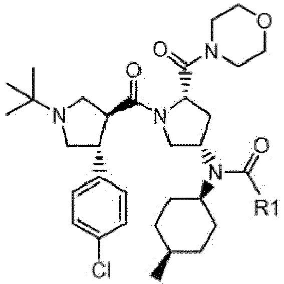 wherein R1 is C₂-C₅ alkyl.

In addition, the present invention provides a pharmaceutical composition for the prevention or treatment of a disease associated with melanocortin receptor having a selective agonistic effect for melanocortin-4 receptor, which comprises a therapeutically effective amount of the compound of Formula 1 or a pharmaceutically acceptable salt thereof, together with a pharmaceutically acceptable carrier.

In addition, the present invention provides a method for the prevention or treatment of a disease associated with melanocortin receptor by selective agonistic effect for melanocortin-4 receptor, which comprises administering a therapeutically effective amount of the compound of Formula 1 or a pharmaceutically acceptable salt thereof to a subject in need thereof.

In addition, the present invention provides use of the compound of Formula 1 or a pharmaceutically acceptable salt thereof as a selective agonist for melanocortin-4 receptor.

The present invention is described in detail hereinafter.

According to one aspect of the present invention, there is provided a medicament for the prevention or treatment of a disease associated with melanocortin receptor having a selective agonistic effect for melanocortin-4 receptor, which comprises a therapeutically effective amount of the compound of Formula 1 or a pharmaceutically acceptable salt thereof.

According to another aspect of the present invention, there is provided a pharmaceutical composition for the prevention or treatment of a disease associated with melanocortin receptor having a selective agonistic effect for melanocortin-4 receptor, which comprises a therapeutically effective amount of the compound of Formula 1 or a pharmaceutically acceptable salt thereof, together with a pharmaceutically acceptable carrier.

In one embodiment according to the present invention, the compound of Formula 1 is *N-*((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*((1*s*,4*R*)-4-methylcyclohexyl)isobutyramide of the following Formula 2:

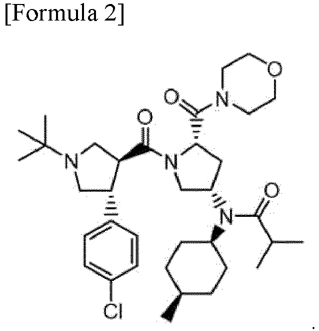

In another embodiment according to the present invention, examples of the pharmaceutically acceptable salt include acid addition salts formed by an inorganic acid such as hydrochloric acid, sulfuric acid, nitric acid, phosphoric acid, hydrobromic acid, and hydroiodic acid; an organic carboxylic acid such as tartaric acid, formic acid, citric acid, acetic acid, trichloroacetic acid, trifluoroacetic acid, gluconic acid, benzoic acid, lactic acid, fumaric acid, and maleic acid; and sulfonic acid such as methanesulfonic acid, benzenesulfonic acid, p-toluenesulfonic acid, and naphthalenesulfonic acid, but is not limited thereto. In another embodiment according to the present invention, the pharmaceutically acceptable salt may be selected from the group consisting of hydrochloric acid, sulfuric acid, nitric acid, phosphoric acid, hydrobromic acid and hydroiodic acid. In another embodiment according to the present invention, the pharmaceutically acceptable salt is hydrochloride.

In another embodiment according to the present invention, the hydrochloride of the compound of Formula 1 may be prepared according to the following Reaction Scheme 1. However, a person skilled in the art may prepare the compound of Formula 1 by various methods based on the structure of Formula 1.

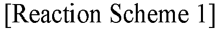

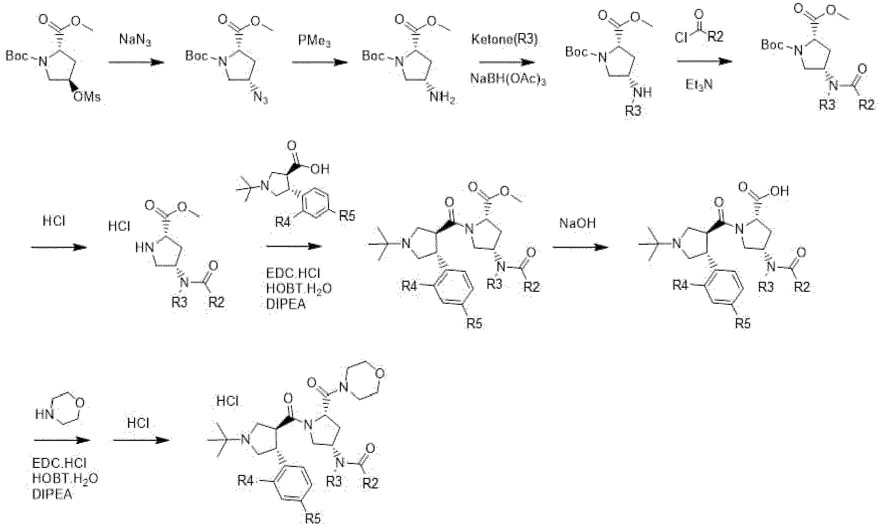

In Reaction Scheme 1,
R2 is C₁-C₅ alkyl;
R3 is C₃-C₈ cycloalkyl unsubstituted or substituted with 1 or 2 C₁-C₅ alkyl; and
R4 and R5 are each independently hydrogen or halogen.

In another embodiment according to the present invention, the selectivity of the compound of Formula 1 or a pharmaceutically acceptable salt thereof for melanocortin-4 receptor may be 2 or more in a ratio to melanocortin-1 receptor. In another embodiment according to the present invention, the selectivity of the compound of Formula 1 or a pharmaceutically acceptable salt thereof for melanocortin-4 receptor may be 3 or more in a ratio to melanocortin-1 receptor. In another embodiment according to the present invention, the selectivity of the compound of Formula 1 or a pharmaceutically acceptable salt thereof for melanocortin-4 receptor may be 4 or more in a ratio to melanocortin-1 receptor. In another embodiment according to the present invention, the selectivity of the compound of Formula 1 or a pharmaceutically acceptable salt thereof for melanocortin-4 receptor may be 5 or more in a ratio to melanocortin-1 receptor.

In another embodiment according to the present invention, the selectivity of the compound of Formula 1 or a pharmaceutically acceptable salt thereof for melanocortin-4 receptor can be confirmed by measuring and comparing the agonistic activities of the receptors. For example, the agonistic activities for melanocortin-4 receptor (MC4R) and melanocortin-1 receptor (MC1R) are measured as EC₅₀ (half maximal effective concentration, the concentration that induces 50% of maximal agonistic activity), and then because in the case of EC₅₀, the lower the value, the better the agonistic ability, it can be confirmed that the larger the value of the ratio, the better the selectivity for MC4R by calculating the ratio of EC₅₀ in MC1R to EC₅₀ in MC4R (MC1R/MC4R).

In another embodiment according to the present invention, the disease associated with melanocortin receptor may be selected from the group consisting of obesity, diabetes, inflammation and erectile dysfunction. In another embodiment according to the present invention, the disease associated with melanocortin receptor may be obesity.

In another embodiment according to the present invention, the medicament or pharmaceutical composition may reduce pigmentation. The compound of Formula 1 or a pharmaceutically acceptable salt thereof according to the present invention has excellent selectivity for melanocortin-4 receptor in comparison to other subtypes of melanocortin receptors-for example, the compound of Formula 1 according to the present invention or a pharmaceutically acceptable salt thereof has excellent selectivity for melanocortin-4 receptor compared to melanocortin-1 receptor, thereby reducing skin pigmentation and effectively preventing or treating diseases associated with melanocortin receptor.

In another embodiment according to the present invention, a "therapeutically effective amount" for an individual subject refers to an amount sufficient to achieve the above pharmacological effect-i.e., the therapeutic effect as described above. The amount of the compound may vary depending on the condition and severity of the subject, the mode of administration and the age of the subject to be treated, but could be determined by persons of ordinary skill in the art based on their knowledge.

In another embodiment according to the present invention, the therapeutically effective dosage of the compound of Formula 1 is, for example, typically in the range of about 0.1 to 500 mg per day according to the frequency and intensity of administration. A typical daily dose of intramuscular or intravenous administration for adults is in the range of about 0.1 to 300 mg per day which can be administered in divided unit dosages. Some patients may need a higher daily dose.

In the present invention, a "pharmaceutical composition" may include other components such as carriers, diluents, excipients, etc., in addition to the active ingredient of the present invention. Accordingly, the pharmaceutical composition may include pharmaceutically acceptable carriers, diluents, excipients or combinations thereof, if necessary. The pharmaceutical composition facilitates the administration of compounds into the body. Various methods for administering the compounds include, but are not limited to, oral, injection, aerosol, parenteral and local administration.

Herein, a "carrier" means a compound that facilitates the addition of compounds into the cell or tissue. For example, dimethylsulfoxide (DMSO) is a conventional carrier facilitating the administration of many organic compounds into living cells or tissues.

Herein, a "diluent" means a compound that not only stabilizes a biologically active form but is diluted in solvent dissolving the compounds. A dissolved salt in buffer is used as a diluent in this field. A conventionally used buffer is a phosphate buffer saline mimicking salt form in body fluid. Since a buffer solution can control the pH of the solution at low concentration, a buffer diluent hardly modifies the biological activity of compounds.

Herein, "pharmaceutically acceptable" means such property that does not impair the biological activity and physical property of compounds.

The compounds according to the present invention can be formulated as various pharmaceutically administered dosage forms. In the preparation of the pharmaceutical composition of the present invention, an active component-specifically, the compound of Formula 1 or a pharmaceutically acceptable salt thereof-is mixed with selected pharmaceutically acceptable carriers considering the dosage form to be prepared. For example, the pharmaceutical composition of the present invention can be formulated as injections, oral preparations and the like, as needed.

The compound of the present invention can be formulated by conventional methods using known pharmaceutical carriers and excipients, and inserted into a unit or multi-unit containers. The formulations may be solution, suspension or emulsion in oil or aqueous solvent and include conventional dispersing agents, suspending agents or stabilizing agents. In addition, the compound may be, for example, dry powder form which is dissolved in sterilized pyrogen-free water before use. The compound of the present invention can be formulated into suppositories by using a conventional suppository base such as cocoa butter or other glycerides. Solid forms for oral administration include capsules, tablets, pills, powders and granules. Capsules and tablets are preferred. Tablets and pills are preferably enteric-coated. Solid forms are manufactured by mixing the compounds of the present invention with at least one carrier selected from inert diluents such as sucrose, lactose or starch, lubricants such as magnesium stearate, disintegrating agents, binders and the like. In addition, it may be formulated as a transdermal dosage form-for example, as a lotion, ointment, gel, cream, patch or spray.

Herein, the term "prevention" refers to reducing or eliminating the possibility of contracting a disease.

Herein, the term "treatment" is used to mean deterring, delaying or ameliorating the progress of diseases in a subject exhibiting symptoms of diseases.

### EFFECTS OF THE INVENTION

The medicament or pharmaceutical composition according to the present invention can effectively prevent or treat diseases associated with melanocortin receptor such as obesity, diabetes, inflammation or erectile dysfunction while ensuring safety without the risk of side effects due to actions on other melanocortin receptors by excellent selectivity for melanocortin-4 receptor.

### BRIEF DESCRIPTION OF DRAWINGS

Figure 1 is a result of measuring the concentration of a drug in the skin and plasma according to repeated administration in a mouse model.
Figure 2 is the result of confirming the pigmentation patterns on the skin and skin hair in a repeated toxicity test in monkeys.

### MODE FOR THE INVENTION

Hereinafter, the present invention is explained in more detail with the following examples. However, it must be understood that the protection scope of the present invention is not limited to the examples.

### Preparation Example: Synthesis of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

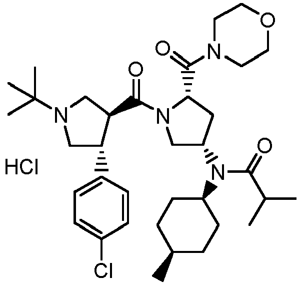

The title compound was obtained through Steps A, B, C and D.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2*S*,4*S*)-4-(*N*-((1*s*,4*R*)-4-methylcyclohexyl)isobutyramido)pyrrolidine-2-carboxylate hydrochloride (28.7 g, 82.73 mmol), (3*S*,4*R*)-1-(*tert*-butyl)-4-(4-chlorophenyl)pyrrolidine-3-carboxylic acid (24.5 g, 86.87 mmol), 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in *N,N'-*dimethylformamide (400 mL), and *N*,*N'*-diisopropylethylamine (72.0 mL, 413.66 mmol) was slowly added. After stirring at room temperature for 16 hours, the reaction solvent was concentrated under reduced pressure, then a 0.5 N aqueous sodium hydroxide solution was added, and extraction was performed twice with ethyl acetate. The organic layer was washed twice with an aqueous sodium chloride solution and water, dried over anhydrous magnesium sulfate, and filtered. The filtrate was concentrated under reduced pressure and purified by column chromatography to obtain the title compound (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2*S*,4*S*)-1-((3*S*,4*R*)-1-(*tert*-butyl)-4-(4-chlorophenyl)pyrrolidine-3-carbonyl)-4-(*N*((1*s*,4*R*)-4-methylcyclohexyl)isobutyramido)pyrrolidine-2-carboxylate (39.4 g, 68.62 mmol) obtained in Step A was dissolved in methanol (450 mL), and a 6N aqueous sodium hydroxide solution (57.2 mL, 343.09 mmol) was added. After stirring at room temperature for 16 hours and adjusting pH to about 5 with a 6 N aqueous hydrochloric acid solution, the reaction solution was concentrated under reduced pressure. The concentrate was dissolved in dichloromethane, and then the insoluble solid was filtered with a paper filter. The filtrate was concentrated under reduced pressure to obtain crude (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3 -carbonyl)-5 -(morpholine-4-carbonyl)pyrrolidin-3 -yl)-N-((1s,4R)-4-methylcyclohexyl)isobutyramide

(2*S*,4*S*)-1-((3*S*,4*R*)-1-(*tert*-butyl)-4-(4-chlorophenyl)pyrrolidine-3-carbonyl)-4-(*N-*((1*s*,4*R*)-4-methylcyclohexyl)isobutyramido)pyrrolidine-2-carboxylic acid (38.4 g, 68.60 mmol) obtained in Step B, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in *N*,*N*'-dimethylformamide (200 mL), and morpholine (5.9 mL, 68.80 mmol) and *N*,*N*'-diisopropylethylamine (59.7 mL, 343.02 mmol) were slowly and sequentially added. After stirring at room temperature for 16 hours, the reaction solution was concentrated under reduced pressure, a 0.5 N aqueous sodium hydroxide solution was added, and extraction was performed twice with ethyl acetate. The organic layer was washed twice with an aqueous sodium chloride solution and water, dried over anhydrous magnesium sulfate, and filtered. The filtrate was concentrated under reduced pressure and purified by column chromatography to obtain the title compound (37.05 g, 86%).
MS [M+H] = 630 (M+1)

### Step D: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide hydrochloride

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide (5.0 g, 7.95 mmol) obtained in Step C was dissolved in ethyl acetate (50 mL), and a 2N hydrochloric acid ethyl acetate solution (3.97 mL, 15.89 mmol) was slowly added. After stirring at room temperature for 30 minutes, the reaction solvent was concentrated under reduced pressure. The resulting crude solid was purified by trituration using hexane and diethyl ether to obtain the title compound (5.23 g, 99%).
MS [M+H] = 630 (M+1)
¹H NMR (500 MHz, CD₃OD) δ 7.49-7.44 (m, 4H), 4.83 (m, 1H), 4.23-4.20 (m, 1H), 3.95-3.91 (m, 2H), 3.79-3.47 (m, 14H), 3.03-3.00 (m, 1H), 2.86-2.82 (m, 1H), 2.73-2.67 (m, 1H), 2.20-2.14 (m, 1H), 1.97 (m, 1H), 1.80-1.62 (m, 5H), 1.50 (s, 9H), 1.44-1.27 (m, 3H), 1.06-1.04 (m, 9H)

### Example 1: Comparison experiment of in vitro activity for MC1R and MC4R

The activities for MC1R and MC4R of *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-A-((1*s*,4*R*)-4-methylcyclohexyl)isobutyramide hydrochloride (hereinafter referred to as "Test Compound") obtained in the Preparation Example, and α-MSH, NDP-α-MSH and MTII (melanotan II)-which are known as melanocortin receptor agonists, as comparative substances-were measured through the following luciferase assay, binding affinity, β-arrestin assay and cAMP assay. The results are represented in Tables 1 to 4, respectively.

### Example 1-1: Luciferase Assay

In order to measure the agonist ability for melanocortin-1 receptor (MC1R) and melanocortin-4 receptor (MC4R), a cell line that permanently expresses MC1R, MC4R and the luciferase gene (CRE-LUC) under the control of CRE (cAMP response element) was established. After a mammalian cell expression vector (pCDNA3 (Neo)) (Invitrogen) containing the MC1R and MC4R genes was prepared, human embryonic kidney (HEK) cell lines were transformed by using Lipofectamine 2000 (Invitrogen) together with a vector (pCRE-Luc)(Stratagen) expressing a luciferase gene (CRE-LUC) under the control of a cAMP response element (CRE). Transformed cell lines (HEK MC1R-Luc and HEK MC4R-Luc) were incubated in a 37°C incubator in the presence of 5% CO₂ for 24 hours by using Dulbecco's Modified Eagles Medium (DMEM) containing 10% heat-inactivated fetal bovine serum (GIBCO/BRL). The cell lines were incubated for four days in the presence of Dulbecco's Modified Eagles Medium (DMEM) containing 10 mL of selection medium (10% heat-inactivated fetal bovine serum (GIBCO/BRL), 100 unit/mL of penicillin (GIBCO/BRL), 100 unit/mL of streptomycin (GIBCO/BRL) and 800 µg/mL of Geneticin (G418) (GIBCO/BRL). The process of removing cells killed by the selection medium by replacing the medium with 10 mL of a new selection medium was repeated three times, once every 4 days. Individual colonies formed by the finally selected and propagated clones were transferred under a microscope to a 24-well cell culture plate containing 1 mL of selection medium per well and incubated for 4 days. Forskolin (SIGMA) was treated to a final concentration of 10 µM, and then incubated for five hours in a 37°C incubator in the presence of 5% CO₂. Each well was treated with 50 µL of a Bright-Glo luciferase reagent (Promega) and left at room temperature for 15 minutes, and then the luminescence of each well was measured by using a luminometer (Victor). Clones exhibiting luminescence of 100 times or more of the basic value by treatment with Forskolin were selected and used to measure the MC1R and MC4R agonist abilities of each compound.

HEK MC1R-Luc cells and HEK MC4R-Luc cells were added to each well of a 96-well luminometer cell culture plate (Costar) to a size of 2.5 × 10⁴ cells in 100 µL of a culture medium and then incubated in a 37°C incubator in the presence of 6% CO₂ for 18 hours. The MCR agonist diluted at each step concentration by using the above culture medium was treated so that the final DMSO concentration did not exceed 1%, and then incubated for five hours in a 37°C incubator in the presence of 6% CO₂. Each well was treated with 50 µL of a Bright-Glo luciferase reagent (Promega) and left at room temperature for five minutes, and then the luminescence of each well was measured by using a luminometer (Victor). The amount of luminescence induced by the agonist diluted at each step concentration was converted into a relative % value with respect to the amount exhibited by a 10 µM NDP-α-MSH treatment. EC₅₀ is expressed as a concentration that induces 50% of the maximum amount of luminescence that can be induced by each agonist. The measurements were measured using statistical software (Prizm).

Table 1 shows the results of measuring the agonist abilities for MC1R and MC4R of each compound obtained by the above experiments in EC₅₀ (nM) units.

**[Table 1]**

| **Luciferase** | **EC₅₀ (nM)** | | **Ratio** |
|---|---|---|---|
| | **MC4R** | **MC1R** | **MC1R/MC4R** |
| Test Compound | 0.434 | 2.501 | 5.76 |
| α-MSH | 17.46 | 0.585 | 0.03 |

### Example 1-2: Binding Affinity

After the CHO-K1 cell line expressing human recombinant MC1R and the HEK-293 cell lines expressing MC4R were established, membranes were collected from each cell line. In a 96-well cell culture plate, the cell line membrane and 25 mM HEPES-KOH adsorption buffer (pH 7.0) containing the MCR agonist diluted at each step concentration were added, and the reaction was carried out. EC₅₀ was calculated as a concentration that induces 50% of the maximum binding that can be induced by each agonist, and the results are represented in Table 2.

**[Table 2]**

| **Binding** | **EC₅₀ (nM)** | | **Ratio** |
|---|---|---|---|
| | **MC4R** | **MC1R** | **MC1R/MC4R** |
| Test Compound | 62 | 310 | 5.00 |
| NDP α-MSH | 0.2 | 0.034 | 0.17 |

### Example 1-3: β-arrestin Assay

A Pathhunter eXpress β-arrestin cell line (U2OS cell line) in which Prolink (PK)-tagged MC1R and MC4R, and enzyme acceptor (EA)-tagged β-arrestin were expressed together was established. When the MCR-PK portion of this cell line is activated, β-arrestin-EA is mobilized, and enzyme acceptor (EA) and Prolink (PK), which are the β-galactosidase enzyme fragments, interact. The activated enzyme hydrolyzes the substrate by β-galactosidase activity to produce a chemiluminescent signal, so that the activity can be measured. After the Pathhunter eXpress β-arrestin cell line (U2OS cell line) was incubated, the cells were inoculated into each well of the cell culture plate and incubated for 48 hours in a 37°C incubator in the presence of 5% CO₂. After the incubation, 5 µL of the sample diluted 5 times with buffer was added, the vehicle concentration was set to 1%, and the MC4R agonist compound diluted at each step concentration was added, followed by reaction at 37°C for 90 minutes. The activity (%) of each agonist compound is expressed as 100% × (average RLU value of sample - average RLU value of vehicle control) / (average maximum value of control ligand - average RLU value of vehicle control), and the value was analyzed by CBIS data analysis suite (ChemInnovation, CA). Table 3 shows the results of measuring the activity ability of the melanocortin receptor of each compound obtained by the above experiments in EC₅₀ (nM) units.

**[Table 3]**

| **β-Arrestin** | **EC₅₀ (nM)** | | **Ratio** |
|---|---|---|---|
| | **MC4R** | **MC1R** | **MC1R/MC4R** |
| Test Compound | 5.14 | 195.23 | 37.98 |
| MTII | 0.2 | 0.17 | 0.85 |
| α-MSH | 23.47 | 0.57 | 0.02 |

### Example 1-4: cAMP Assay

After cAMP Hunter Gs-coupled cell lines (CHO-K1 cell line) in which each of MC1R and MC4R was overexpressed were established so that the increase in the cAMP level in cells due to agonist reaction was measurable, the cells were inoculated into each well of a white cell culture plate and incubated for 24 hours in a 37°C incubator in the presence of 5% CO₂. After the incubation, the medium was removed, and 15 µL of a 2:1 HBBS/10 mM HEPES:cAMP XS+Ab reagent was added. After 5 µL of the sample diluted 4 times with buffer was added, the vehicle concentration was set to 1%, and the MC4R agonist compound diluted at each step concentration was added, followed by reaction at 37°C for 30 minutes. The activity (%) of each agonist compound is expressed as 100% × (average RLU value of sample - average RLU value of vehicle control) / (average RLU value of max control - average RLU value of vehicle control), and the value was analyzed by CBIS data analysis suite (ChemInnovation, CA). Table 4 shows the results of measuring the agonist ability of melanocortin receptors of the compounds obtained by the above experiments in EC₅₀ (nM) units.

**[Table 4]**

| **cAMP** | **EC₅₀ (nM)** | | **Ratio** |
|---|---|---|---|
| | **MC4R** | **MC1R** | **MC1R/MC4R** |
| Test Compound | 16.28 | 84.82 | 5.21 |
| MTII | 3.35 | 0.86 | 0.26 |
| α-MSH | 9.62 | 0.78 | 0.08 |

In Tables 1 to 4, the lower the EC₅₀ value, the better the agonistic ability for the corresponding receptor. Specificity for MC4R can be confirmed by calculating the ratio of EC₅₀ in MC1R to EC₅₀ in MC4R, and a larger value of the ratio indicates superior selectivity for MC4R. The above ratio of the Test Compound according to the present invention is from at least 5 to a maximum of 37.98. From this, it was confirmed that the Test Compound of the present invention exhibits excellent selectivity for MC4R involved in energy metabolism and body weight control *in vivo* compared to MC1R involved in skin pigmentation.

### Example 2: Measurement of drug concentration and distribution in the skin

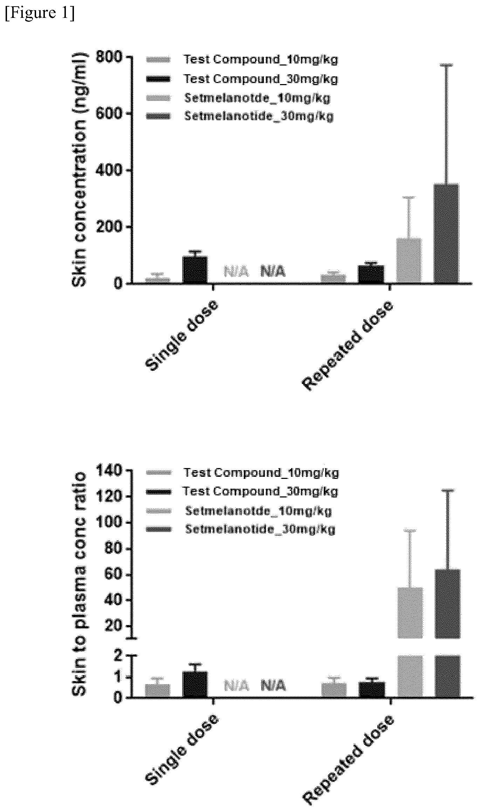

With respect to skin pigmentation-which is an off-target effect of MC4R agonist, the distribution and accumulation of the drug were evaluated by measuring the drug concentration in the skin and plasma after repeated administration in a db/db mouse model lacking leptin receptor. The results are represented in Figure 1.

As a comparative substance, setmelanotide (Ac-RC[dA]H[dF]RWC-NH₂ (disulfide) (AcOH salt))-which is an MC4R agonist peptide with low selectivity for MC4R compared to MC1R and which is reported to show skin pigmentation in clinical trials (Clement et al., Nature Medicine, 2018)-was used.

As can be seen from Figure 1, it was confirmed that the Test Compound of the present invention did not relatively accumulate in the skin after repeated administration.

### Example 3: Measurement of skin pigmentation

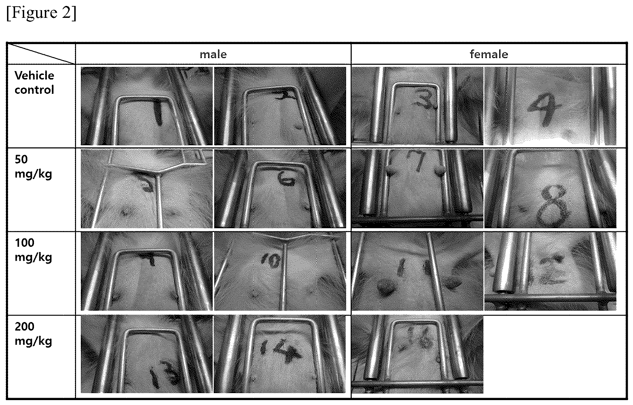

In order to confirm the possibility of occurrence of skin pigmentation-which is an off-target effect of MC4R agonist, pigmentation patterns in the skin and skin hair were measured in a repeated toxicity test in monkeys, and the results are represented in Figure 2.

As can be seen from Figure 2, in the case of the Test Compound of the present invention, it was confirmed that pigmentation did not appear on the skin and skin hair when repeatedly administered for 2 weeks.

## Claims

1. A medicament for the prevention or treatment of a disease associated with melanocortin receptor having a selective agonistic effect for melanocortin-4 receptor, which comprises a therapeutically effective amount of a compound of the following Formula 1 or a pharmaceutically acceptable salt thereof: 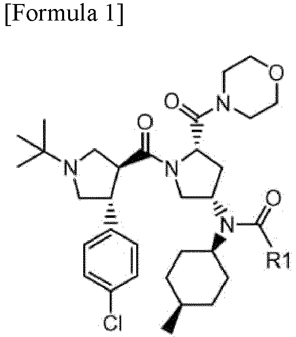 wherein R1 is C₂-C₅ alkyl.

2. The medicament according to Claim 1, wherein the compound of Formula 1 is *N-*((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 2:

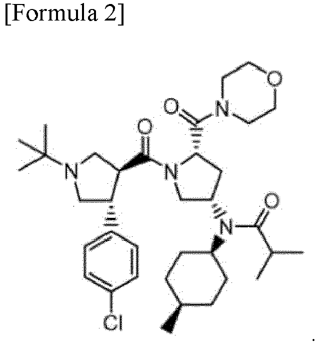

3. The medicament according to Claim 1, wherein the pharmaceutically acceptable salt is selected from the group consisting of hydrochloric acid, sulfuric acid, nitric acid, phosphoric acid, hydrobromic acid and hydroiodic acid.

4. The medicament according to Claim 1, wherein the selectivity of the compound of Formula 1 or a pharmaceutically acceptable salt thereof for melanocortin-4 receptor is 2 or more in a ratio to melanocortin-1 receptor.

5. The medicament according to Claim 4, wherein the selectivity of the compound of Formula 1 or a pharmaceutically acceptable salt thereof for melanocortin-4 receptor is 5 or more in a ratio to melanocortin-1 receptor.

6. The medicament according to Claim 1, wherein the disease associated with melanocortin receptor is selected from the group consisting of obesity, diabetes, inflammation and erectile dysfunction.

7. The medicament according to Claim 6, wherein the disease associated with melanocortin receptor is obesity.

8. The medicament according to Claim 1, wherein pigmentation is reduced.

9. The medicament according to Claim 1, which is an oral formulation.

10. A pharmaceutical composition for the prevention or treatment of a disease associated with melanocortin receptor having a selective agonistic effect for melanocortin-4 receptor, which comprises a therapeutically effective amount of a compound of the following Formula 1 or a pharmaceutically acceptable salt thereof, together with a pharmaceutically acceptable carrier: 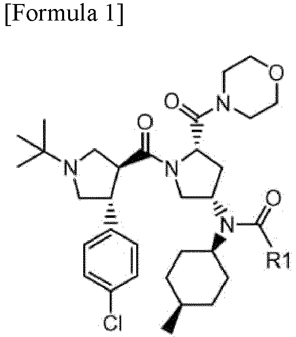 wherein R1 is C₂-C₅ alkyl.

11. The pharmaceutical composition according to Claim 10, wherein the compound of Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 2:

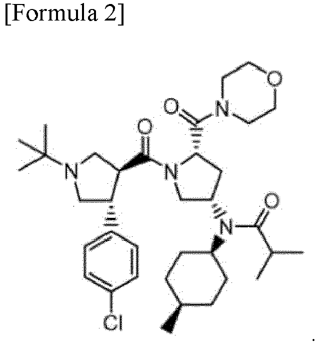

12. The pharmaceutical composition according to Claim 10, wherein the pharmaceutically acceptable salt is selected from the group consisting of hydrochloric acid, sulfuric acid, nitric acid, phosphoric acid, hydrobromic acid and hydroiodic acid.

13. The pharmaceutical composition according to Claim 10, wherein the selectivity of the compound of Formula 1 or a pharmaceutically acceptable salt thereof for melanocortin-4 receptor is 2 or more in a ratio to melanocortin-1 receptor.

14. The pharmaceutical composition according to Claim 13, wherein the selectivity of the compound of Formula 1 or a pharmaceutically acceptable salt thereof for melanocortin-4 receptor is 5 or more in a ratio to melanocortin-1 receptor.

15. The pharmaceutical composition according to Claim 10, wherein the disease associated with melanocortin receptor is selected from the group consisting of obesity, diabetes, inflammation and erectile dysfunction.

16. The pharmaceutical composition according to Claim 15, wherein the disease associated with melanocortin receptor is obesity.

17. The pharmaceutical composition according to Claim 10, wherein pigmentation is reduced.

18. The pharmaceutical composition according to Claim 10, which is an oral formulation.
